# EUROPEAN PATENT APPLICATION

(11) **EP 4 169 934 A1**
(43) Date of publication of application: **26.04.2023**
(21) Application number: 21825273.2
(22) Date of filing: 18.06.2021
(51) Int. Cl.: C07K 14/315, A61K 39/09, A61K 39/40, C07K 16/12

(54) **MUTANT OF IMMUNOGLOBULIN DEGRADING ENZYME IDEE**

(30) Priority: 18.06.2020 CN 202010557830
(71) Applicant: Shanghai Bao Pharmaceuticals Co., Ltd., Shanghai 201908 (CN)
(72) Inventor: LIU, Yanjun, Shanghai 201908 (CN); WANG, Zheng, Shanghai 201908 (CN)
(74) Representative: Pharma Patents International AG
(86) International application number: PCT/CN2021/100844
(87) International publication number: WO 2021/254479

(57) **Abstract**

Provided is a mutant of an immunoglobulin degrading enzyme IdeE. The immunoglobulin degrading enzyme IdeE includes an amino acid sequence as shown in SEQ ID NO: 2 in the sequence listing. The mutant is obtained by means of replacing at least one or more of the positions 8, 10, 24, 59, 97, and 280 of the amino acid sequence. The function of the mutant includes at least the function of the immunoglobulin degrading enzyme IdeE. The activity and thermal stability of the provided mutant of the immunoglobulin degrading enzyme are higher than those of a wild-type IdeE.

## Description

### Technical Field

The present invention relates to the field of biotechnology, in particular to a mutant of an immunoglobulin-degrading enzyme.

### Background Art

*Streptococcus pyogenes* is one of the common pathogens in humans and animals. It is widely found in nature and in the oropharyngeal cavity, respiratory tract and intestinal tract of humans or animals. Streptococcal infection may induce related diseases, from milder diseases such as pyogenic dermatitis and pharyngitis, to more serious diseases such as sepsis, necrotizing fasciitis and toxic shock syndrome. Immunoglobulin G-degrading enzyme of *Streptococcus pyogenes* (IdeS), a common Group A *Streptococcus pyogenes* (GAS) cysteine protease, has endopeptidase activity that hydrolyzes IgG (Agniswamy J, Lei B, Musser J M et al., J Biol Chem, 2004, 279: 52789-52796. Lei B, DeLeo F R, Reid S D et al., Infect Immun, 2002, 70: 6880-6890. Von Pawel-Rammingen U, Johansson B P, Bjorck L. EMBO J, 2002, 21: 1607-1615.). As a virulence factor of pathogens, it can recognize the CH1 and CH2 domains in the lower hinge regions of antibodies and specifically degrade IgG, to obtain homogeneous F(ab)₂ and Fc fragments, helping GASs to evade antibody-mediated phagocytosis and cytotoxicity, thus weakening the killing of GASs by the host's immune system (Von Pawel-Rammingen U. J Innate Immunity, 2012, 4: 132-140. Su, Y.-F. et al., Molecular Immunology, 2011, 49: 134-142.).

Immunoglobulin G (IgG) is the main antibody component in serum, and accounts for about 75% of serum immunoglobulins. It mainly plays a protective role in the body's immunity and can effectively prevent infectious diseases. In addition to its protective effect, IgG is also related to diseases. In some autoimmune diseases, anti-IgG antibodies react with the body's own molecules, and IgG may cause acute transplant rejection in organ transplantation. IdeS specifically degrades IgG, causing IgG to lose its inherent function, thus achieving immunosuppression.

At present, IdeS used in clinical practice has the problems of poor activity and high pre-existing antibodies in the human body. IdeS is a virulence factor of human pathogens. Clinical studies have found that anti-IdeS antibodies are detected nearly 100% in normal people under normal physiological conditions, which leads to low administration efficiency and safety concerns in the use of IdeS.

Protease IdeE with a sequence homology of about 70% to IdeS is derived from a *Streptococcus equi* subsp. zooepidemicus - *Streptococcus equi* ssp. equi, an equine pathogen (Jonas Lannergård, Bengt Guss. FEMS Microbiol Lett, 2006, 262: 230-235). The two enzymes, IdeE and IdeS, cleave IgG at exactly the same position. The cleavages are highly reproducible and specific, and have a very similar substrate range. Since IdeE is derived from an equine pathogen, it is speculated that its pre-existing antibodies in the human body may be much lower than IdeS, and it is more suitable for the development of immunosuppressants for treating and preventing diseases mediated by anti-IgG antibodies. However, wild-type IdeE, like IdeS, also has the problem of low activity. Therefore, it is necessary to improve the activity of IdeE through molecular design and mutation screening, reducing doses used in clinical practice, thus reducing the risks posed by high doses of proteins of bacterial origin. This is exactly what the present invention aims to achieve.

### Summary of the Invention

The first aspect of the present invention relates to a mutant of an immunoglobulin-degrading enzyme IdeE, wherein the immunoglobulin-degrading enzyme IdeE comprises or consists of an amino acid sequence as set forth in SEQ ID NO: 2 in the Sequence Listing; and the mutant comprises a mutation selected from the group consisting of:
(1) substitution of one or more of positions 8, 10, 24, 59, 97 and 280 of the amino acid sequence, thereby obtaining the mutant; and/or
(2) truncation of the immunoglobulin-degrading enzyme IdeE, by deleting the sequence of the first 1, the first 2, the first 3, the first 4, the first 5, the first 6, the first 7, the first 8, the first 9, the first 10, the first 11, the first 12, the first 13, the first 14, the first 15, the first 16, the first 17, the first 18 or the first 19 amino acids at its N-terminus; and/or
(3) truncation of the immunoglobulin-degrading enzyme IdeE, by deleting the sequence of the last 1, the last 2, the last 3, the last 4, the last 5, the last 6, the last 7, the last 8, the last 9 or the last 10 amino acids at its C-terminus;
wherein the mutant has higher activity and/or thermal stability than the activity and/or thermal stability of the immunoglobulin-degrading enzyme IdeE.

The second aspect of the present invention relates to a protein comprising the mutant of the present invention. The protein is linked to a secretory signal sequence and/or methionine at the N-terminus of the mutant; and/or the protein is linked to a histidine tag at the C-terminus of the mutant.

The third to fifth aspects of the present invention relate to a nucleotide encoding the mutant or protein of the present invention, an expression vector comprising the nucleotide, and a host cell comprising the expression vector or expressing the mutant or protein of the present invention.

The sixth aspect of the present invention relates to a composition or kit, comprising: the mutant or protein of the present invention; and optionally, a substance selected from the group consisting of: a pharmaceutically acceptable carrier or excipient, an antibody or an Fc-containing protein and a viral vector drug.

### Brief Description of the Drawings

Figure 1 shows the SDS-PAGE gel electrophoresis pattern of cleavage products resulting from cleavage of human IgG 1 by seven single-point mutants and wild-type IdeE (enzyme : substrate = 1 : 1000).
Figure 2 shows the SDS-PAGE gel electrophoresis pattern of cleavage products resulting from cleavage of human IgG 1 by seven single-point mutants and wild-type IdeE (enzyme : substrate = 1 : 2000).
Figure 3 shows the SDS-PAGE gel electrophoresis pattern of cleavage products resulting from cleavage of human IgG1 by five N-terminal truncated mutants (enzyme : substrate = 1 : 1000).
Figure 4 shows the SDS-PAGE gel electrophoresis pattern of cleavage products resulting from cleavage of human IgG1 by five N-terminal truncated mutants and wild-type IdeE after being placed at 50°C for 1 h (enzyme : substrate = 1 : 1000).
Figure 5 shows the SDS-PAGE gel electrophoresis pattern of cleavage products resulting from cleavage of human IgG1 by two C-terminal truncated mutants (enzyme : substrate = 1 : 1000).
Figure 6 shows the SDS-PAGE gel electrophoresis pattern of cleavage products resulting from cleavage of human IgG1 by five combinatorial mutants (enzyme : substrate = 1 : 2000).
Figure 7 shows the SDS-PAGE gel electrophoresis pattern of cleavage products resulting from cleavage of human IgG1 by five combinatorial mutants after being placed at 50°C for 1 h (enzyme : substrate = 1 : 2000).
Figure 8 shows the SDS-PAGE gel electrophoresis pattern of cleavage products resulting from cleavage of human IgG1 by mutant E97D_del18 and IdeS at different concentrations.
Figure 9 shows the SDS-PAGE gel electrophoresis pattern of cleavage products resulting from cleavage of human IgG1 by mutant E97D_del18 and IdeZ at different concentrations.
Figure 10 shows the SDS-PAGE gel electrophoresis pattern of cleavage products resulting from cleavage of human IVIg by mutant E97D_del18 in the sera and plasmas of mice.
Figure 11 shows the SDS-PAGE gel electrophoresis pattern of cleavage products produced by mutant E97D_del18 in the sera of mice and humans.
Figures 12A-12D show the SDS-PAGE gel electrophoresis patterns of cleavage products resulting from cleavage of IgG by E97D_del18 at different concentrations in the sera of beagles, rats, mice, rabbits, monkeys and pigs.
Figure 13 shows the SDS-PAGE gel electrophoresis pattern of cleavage products resulting from cleavage of human IVIg by E97D_del18 at different times in mice.
Figures 14A and 14B show the SDS-PAGE gel electrophoresis patterns of cleavage products resulting from cleavage of human IgG1 by mutants with different mutation combinations and IdeE (enzyme : substrate = 1 : 2000).

### Detailed Description of Embodiments

### I. Functional polypeptides with immunoglobulin-degrading enzyme activity

In the first aspect of the present invention, there is provided a functional polypeptide with the activity of immunoglobulin-degrading enzyme, comprising a mutant based on an amino acid sequence as set forth in SEQ ID NO: 2, wherein the mutant is selected from the group consisting of:
(1) a mutant obtained by substituting one or more amino acids at positions 8, 10, 24, 59, 97 and 280 of SEQ ID NO: 2; and/or
(2) an N-terminal truncated mutant of SEQ ID NO: 2, selected from those obtained by deleting the sequence of the first 1, the first 2, the first 3, the first 4, the first 5, the first 6, the first 7, the first 8, the first 9, the first 10, the first 11, the first 12, the first 13, the first 14, the first 15, the first 16, the first 17, the first 18 or the first 19 amino acids at its N-terminus; and/or
(3) a C-terminal truncated mutant of SEQ ID NO: 2, selected from those obtained by deleting the sequence of the last 1, the last 2, the last 3, the last 4, the last 5, the last 6, the last 7, the last 8, the last 9 or the last 10 amino acids at its C-terminus.

The mutant of the present invention has the function of the immunoglobulin-degrading enzyme IdeE, and preferably further has improved IgG-cleaving activity and thermal stability.

The term "having higher activity than that of the immunoglobulin-degrading enzyme IdeE" in the present invention refers to the ability of the mutant to degrade immunoglobulins over that of the wild-type immunoglobulin-degrading enzyme IdeE.

The term "having higher thermal stability than that of IdeE" in the present invention refers to the ability of the mutant after being maintained at a certain temperature for a period of time to degrade immunoglobulins over that of the wild-type immunoglobulin-degrading enzyme IdeE under the same conditions.

The mutant of the present invention is preferably produced by means of genetic engineering recombination.

Preferably, the mutant has at least 60%, at least 65%, at least 70%, at least 75%, at least 80%, at least 85%, at least 90%, at least 91%, at least 92%, at least 93%, at least 94%, at least 95%, at least 96%, at least 97%, at least 98% or at least 99% identity to the sequence as set forth in SEQ ID NO: 2.

More preferably, the amino acid at position 8, 10, 24, 59, 97 or 280 is substituted, for example, the amino acid sequence of the resulting mutant is as set forth in any one of SEQ ID NOs: 3-17 and SEQ ID NO: 35;
alternatively, the first 15, the first 16, the first 17, the first 18 or the first 19 amino acids are deleted at the N-terminus of the immunoglobulin-degrading enzyme IdeE, for example, the amino acid sequence of the resulting mutant is as set forth in any one of SEQ ID NOs: 18-22;
alternatively, the last 1, the last 5, the last 8 or the last 10 amino acids are deleted at the C-terminus of the immunoglobulin-degrading enzyme IdeE, for example, the amino acid sequence of the resulting mutant is as set forth in any one of SEQ ID NOs: 23-24;
alternatively, the amino acid at position 8, 10, 24, 59, 97 or 280 is substituted, and the first 15, the first 16, the first 17, the first 18 or the first 19 amino acids, preferably the first 18 amino acids, are deleted at the N-terminus of the immunoglobulin-degrading enzyme IdeE at the same time, for example, the amino acid sequence of the resulting mutant is as set forth in any one of SEQ ID NOs: 25-29;
alternatively, the amino acid at position 8, 10, 24, 59, 97 or 280 is substituted, the first 15, the first 16, the first 17, the first 18 or the first 19 amino acids, preferably the first 18 amino acids, are deleted at the N-terminus of the immunoglobulin-degrading enzyme IdeE at the same time, and the last 1, the last 5, the last 8 or the last 10 amino acids, preferably the last 5 amino acids, are deleted at the C-terminus of the immunoglobulin-degrading enzyme IdeE at the same time, for example, the amino acid sequence of the resulting mutant is as set forth in any one of SEQ ID NOs: 30-34.

In a preferred embodiment of the present invention, the amino acid substitution is selected from the group consisting of:
(1) the threonine at position 8 of SEQ ID NO: 2 is substituted with any one of cysteine, phenylalanine, tryptophan, tyrosine, aspartic acid, glutamic acid, alanine, glycine, histidine, isoleucine, leucine, methionine, asparagine, proline, glutamine, serine, valine, arginine and lysine;
(2) the alanine at position 10 of SEQ ID NO: 2 is substituted with any one of cysteine, aspartic acid, glutamic acid, phenylalanine, glycine, histidine, isoleucine, lysine, leucine, methionine, asparagine, proline, glutamine, arginine, serine, threonine, valine, tryptophan and tyrosine;
(3) the threonine at position 24 of SEQ ID NO: 2 is substituted with any one of alanine, cysteine, aspartic acid, asparagine, glutamic acid, phenylalanine, glycine, histidine, isoleucine, lysine, leucine, methionine, proline, glutamine, arginine, serine, valine, tryptophan and tyrosine;
(4) the alanine at position 59 of SEQ ID NO: 2 is substituted with any one of cysteine, aspartic acid, glutamic acid, phenylalanine, glycine, histidine, isoleucine, lysine, leucine, methionine, asparagine, proline, glutamine, arginine, serine, threonine, valine, tryptophan and tyrosine;
(5) the glutamic acid at position 97 of SEQ ID NO: 2 is substituted with any one of alanine, cysteine, aspartic acid, phenylalanine, glycine, histidine, isoleucine, lysine, leucine, methionine, asparagine, proline, glutamine, arginine, serine, threonine, valine, tryptophan and tyrosine; and
(6) the arginine at position 280 of SEQ ID NO: 2 is substituted with any one of alanine, aspartic acid, glutamic acid, cysteine, serine, phenylalanine, histidine, isoleucine, lysine, leucine, methionine, asparagine, proline, glutamine, threonine, valine, tryptophan and tyrosine.

In a more preferred embodiment of the present invention, the amino acid substitution is selected from the group consisting of:
(1) the threonine at position 8 of SEQ ID NO: 2 is substituted with aspartic acid, glutamic acid, tryptophan or tyrosine;
(2) the alanine at position 10 of SEQ ID NO: 2 is substituted with lysine or arginine;
(3) the threonine at position 24 of SEQ ID NO: 2 is substituted with alanine, glycine or serine;
(4) the alanine at position 59 of SEQ ID NO: 2 is substituted with isoleucine, leucine or valine;
(5) the glutamic acid at position 97 of SEQ ID NO: 2 is substituted with asparagine; and/or
(6) the arginine at position 280 of SEQ ID NO: 2 is substituted with histidine or lysine.

In another preferred embodiment, based on the five sequences of SEQ ID NO: 9, SEQ ID NO: 13, SEQ ID NO: 14, SEQ ID NO: 1, SEQ ID NO: 5 and SEQ ID NO: 16 obtained through amino acid substitution, the first 18 amino acids at their N-termini are further deleted, and the amino acid sequences of the resulting mutants are as set forth in SEQ ID NOs: 25-29 in the Sequence Listing.

In another preferred embodiment, based on the five sequences of SEQ ID NOs: 26-29 obtained through amino acid substitution, the 5 or 10 amino acids at their C-termini are further deleted, and the amino acid sequences of the resulting mutants are as set forth in SEQ ID NOs: 30-34 in the Sequence Listing.

In another preferred embodiment, the combinatorial mutation is further performed based on the three mutants of SEQ ID NOs: 14-16, and the amino acid sequence of the resulting mutant is as set forth in SEQ ID NO: 35 in the Sequence Listing. In another preferred embodiment, based on the five sequences of SEQ ID NO: 9, SEQ ID NO: 13, SEQ ID NO: 14, SEQ ID NO: 1, SEQ ID NO: 5 and SEQ ID NO: 16 obtained through amino acid substitution, the first 18 amino acids at their N-termini are further deleted, and the amino acid sequences of the resulting mutants are as set forth in SEQ ID NOs: 25-29 in the Sequence Listing.

Preferably, the mutant of the present invention can also be further mutated, and the sequence of the variant obtained through further mutation has at least 60%, at least 65%, at least 70%, at least 75%, at least 80%, at least 85%, at least 90%, at least 91%, at least 92%, at least 93%, at least 94%, at least 95%, at least 96%, at least 97%, at least 98% or at least 99% identity to the sequence of SEQ ID NO: 2, and the variant also has the function of the immunoglobulin-degrading enzyme IdeE.

The complete sequence of the IdeE used in the present invention is publicly available under the GenBank accession number ABF57910.1, and its sequence is provided herein as SEQ ID NO: 1. The sequence comprises an N-terminal methionine, followed by a 33-amino acid secretory signal sequence, followed by an IdeE coding sequence. The N-terminal methionine and the signal sequence are usually removed to form a mature IdeE protein, the sequence of which is provided herein as SEQ ID NO: 2. Unless otherwise stated, all references to the numbers of the amino acid positions in the sequences of the immunoglobulin-degrading enzymes disclosed herein are based on the numbers of the corresponding positions in SEQ ID NO: 2 starting from the N-terminus.

The present invention also provides a protein comprising the mutant as described above.

In a preferred embodiment, the protein comprises a signal peptide at the N-terminus of the mutant described above; preferably, the protein is linked to a secretory signal sequence at the N-terminus of the mutant, and to methionine at the N-terminus of the secretory sequence, and/or is linked to a histidine tag at the C-terminus of the mutant; more preferably, the protein comprises or consists of the following from the N-terminus to the C-terminus: methionine, the secretory signal sequence and the mutant.

In the second aspect of the present invention, there is provided a nucleotide encoding the protein or mutant as described above.

The present invention also provides an expression vector comprising the nucleotide.

The present invention also provides a host cell comprising the expression vector as described above, or a host cell expressing the protein or mutant as described above.

The host cell can be a conventional cell for expressing proteins or polypeptides in the art, and is selected from *E. coli* cells, yeast cells, etc.

### II. Pharmaceutical combinations

In the third aspect of the present invention, there is provided a composition, comprising an immunoglobulin-degrading enzyme or a mutant thereof or a protein comprising an immunoglobulin-degrading enzyme or a mutant thereof; and optionally, a pharmaceutically acceptable carrier or excipient. In a specific embodiment, the immunoglobulin-degrading enzyme is selected from IdeE, IdeS and IdeZ. In a specific embodiment, the mutant of the immunoglobulin-degrading enzyme is a mutant as described above, and the protein is a protein comprising the mutant as described above. In a specific embodiment, the composition of the present invention further comprises: an antibody or an Fc-containing protein. In a specific embodiment, the antibody target is selected from the group consisting of: a cell surface protein, a cytokine, a hormone, an enzyme, an intracellular messenger, an intercellular messenger and an immune checkpoint. In a specific embodiment, the composition of the present invention further comprises: a viral vector drug, preferably selected from the group consisting of: an oncolytic virus, a gene therapy virus and a viral vector vaccine. In a specific embodiment, the composition of the present invention further comprises: an agent capable of reducing the IgG level in the blood, preferably selected from the group consisting of: an FcRn antibody and an Fc fragment variant with a high affinity to FcRn.

### 2.1. Antibody targets

Preferably, in the composition as described above, the antibody target can be a cell surface protein, including but not limited: AFP, αv integrin, α4β7 integrin, BCMA, CD2, CD3, CD19, CD20, CD22, CD25, CD30, CD32, CD33, CD36, CD40, CD46, CD52, CD56, CD64, CD70, CD74, CD79, CD80, CD86, CD105, CD121, CD123, CD133, CD138, CD174, CD205, CD227, CD326, CD340, CEA, c-Met, Cripto, CA1X, Claudin18.2, ED-B, EGFR, EpCAM, EphA2, EphB2, FAP, FOLR1, GD2, Globo H, GPC3, GPNMB, HER-1, HER-2, HER-3, MAGE-A3, mesothelin, MUC16, GPNMB, PSMA, TMEFF2, TAG-72, 5T4, ROR-1, Sca-1, SP, VEGF or WT1.

The antibody target can be a cytokine, including but not limited to: interleukins IL-1 to IL-13, tumor necrosis factors α and β, interferons α, β and γ, tumor growth factor β (TGF-β), colony stimulating factor (CSF) or granulocyte-monocyte colony stimulating factor (GM-CSF). See Human Cytokines: Handbook for Basic & Clinical Research (Aggrawal et al. (eds), Blackwell Scientific, Boston, MA 1991).

The antibody target can be a hormone, an enzyme, and an intracellular and intercellular messenger, such as adenylate cyclase, guanylate cyclase or phospholipase C.

The antibody target can be an immune checkpoint, including: CTLA-4, PD-1, PD-L1, TIM-3, LAG3, Siglec15, 4-1BB, GITR, OX40, CD40L, CD28, TIGIT and VISTA.

### 2.2. Targeted drugs

Preferably, in the composition as described above, the combination further comprises a targeted drug or a chemotherapeutic drug or an immune checkpoint blocker, wherein the targeted drug is selected from an epigenetic drug, such as a histone deacetylase inhibitor, an inhibitor targeting the PI3K/Akt/mTOR signaling pathway, such as Tricibine, and a tyrosine kinase inhibitor, such as sunitinib; the chemotherapeutic drug is selected from an immunosuppressant, such as cyclophosphamide, thalidomide and pomalidomide, a proteasome inhibitor, such as bortezomib, a cytotoxic drug, such as gemcitabine and temozolomide, and a cell cycle non-specific drug, such as mitoxantrone; and the immune checkpoint blocker is selected from an anti-CTLA-4 antibody, an anti-PD-1 antibody, an anti-TIM-3 antibody, an anti-LAG3 antibody, an anti-Siglec15 antibody, an anti-4-1BB antibody, an anti-GITR antibody, an anti-OX40 antibody, an anti-CD40L antibody, an anti-CD28 antibody, an anti-TIGIT antibody and an anti-VISTA antibody.

### 2.3. Agents capable of reducing blood IgG level

Preferably, in the composition as described above, the polypeptide drug capable of reducing the IgG level in the blood can block the binding of IgG in the blood to the FcRn protein. Preferably, the affinity of the polypeptide to human FcRn protein is higher than that of IgG in the blood to human FcRn protein; and the IgG is selected from IgG1, IgG2, IgG3 and IgG4. Preferably, the polypeptide comprises an antibody Fc fragment variant, which comprises a mutation capable of increasing the affinity of Fc to FcRn, preferably YTE, YTEKF, LS or NHS, wherein the antibody Fc fragment is, for example, Efgartigimod. The variant can be a monomer, a dimer or a multimer. The positions of the mutations of YTE, YTEKF, LS, NHS, etc., which can be used in the present invention, are as described by Dall'Acqua *et al.* (WF, D. A. et al. (2002). Journal of immunology (Baltimore, Md.: 1950) 169(9): 5171-5180.) and Lee et al. (Lee, C. H. et al. (2019). Nat Commun 10(1): 5031.), respectively. The mutation is performed on one selected from human IgG, which is selected from IgG1, IgG2, IgG3 and IgG4.

Other Fc fragment variants, which can be used in the present invention, comprise mutations including but not limited to those as described by Dall'Acqua *et al.* (WF, D. A. et al. (2002). Journal of immunology (Baltimore, Md.: 1950) 169(9): 5171-5180.), Shan *et al.* (Shan, L. et al. (2016). PLoS One 11(8): e0160345.), Lee *et al.* (Lee, C. H. et al. (2019). Nat Commun 10(1): 5031.), Mackness *et al.* (Mackness, B. C. et al. (2019). MAbs 11(7): 1276-1288.) and Christophe *et al.* (Dumet Christophe, Pottier Jérémy, Gouilleux-Gruart Valérie et al., MAbs, 2019, 11: 1341-1350.).

Preferably, the polypeptide comprises an antibody Fc fragment variant, which comprises a mutation capable of increasing the affinity of Fc to FcyR, preferably S239D/I322E, S239D/I322E/A330L, K326W/E333S or R214K mutation, preferably afucosylation modification. The variant can be a monomer, a dimer or a multimer. Other Fc fragment variants, which can be used in the present invention, comprise mutations including but not limited to those as described by Wang *et al.* (Wang Xinhua., Mathieu Mary., Brezski Randall J. (2018). Protein Cell, 9(1), 63-73. doi: 10.1007/s13238-017-0473-8).

Preferably, the variant comprising a mutation capable of increasing the affinity of Fc to FcRn also comprises a mutation capable of increasing the affinity of Fc to FcyR. The variant can be a monomer, a dimer or a multimer.

Preferably, in the pharmaceutical combination as described above, the polypeptide is selected from an anti-FcRn antibody, such as Nipocalimab, Rozanolixizumab, RVT-1401, HBM9161, ALXN1830, SYNT001 or Nirsevimab.

Preferably, in the pharmaceutical combination as described above, the polypeptide is selected from a small peptide fragment capable of specifically binding to FcRn and with a length of 10-70 amino acids, such as ABY-039.

Preferably, the polypeptide is selected from an Fc multimer capable of specifically binding to FcRn, such as GL-2045, M230, PRIM, HexaGard^{™}, CSL777 or hexavalent molecules by UCB.

Preferably, the polypeptide includes, but is not limited to, a polypeptide fragment as described by Sockolosky *et al.* (Sockolosky Jonathan T, Szoka Francis C. Adv. Drug Deliv. Rev., 2015, 91: 109-24).

### 2.4. Viral vector drugs

Preferably, in the viral vector drug of the composition as described above, the virus used in the viral vector drug is selected from an ssDNA virus, a dsDNA virus, an ssRNA virus or a dsRNA virus; and/or the virus used in the viral vector drug is selected from a wild-type virus strain or naturally attenuated strain, a genetically engineered and selective attenuated strain, a gene-loaded virus strain and a gene transcription-targeting virus strain.

Preferably, the wild-type virus strain or naturally attenuated strain is selected from Newcastle disease virus, reovirus, mumps virus, West Nile virus, adenovirus, vaccinia virus, etc.

Preferably, the genetically engineered and selective attenuated strain enables the virus to selectively replicate in a tumor by manually deleting a key gene, such as thymidine kinase (TK)-knockout genetically modified human herpes simplex virus I (HSV-1), and the genetically engineered and selective attenuated strain is, for example, ONYX-015 or G207. In ONYX-015, a segment of 827 bp in the E1b region is deleted, and a point mutation is made at the gene for protein E1B55K, so that its expression is terminated prematurely, and the E1B55K protein can not be expressed. In G207, the y34.5 gene is deleted, which is the determinant of HSV-1 neurotoxicity.

Preferably, the gene-loaded virus strain is loaded with an exogenous gene, such as one of granulocyte-macrophage colony stimulating factor (GM-CSF), and the gene-loaded virus strain is, for example, JX-594 or T-VEC.

Preferably, the gene transcription-targeting virus strain enables to control the replication of the oncolytic virus in a tumor cell by inserting a tissue- or tumor-specific promoter upstream of an essential gene of the virus, and the gene transcription-targeting virus strain is, for example, G92A.

Preferably, in the pharmaceutical combination as described above, the ssDNA virus is selected from parvovirus, preferably H-1PV virus.

Preferably, the dsDNA virus is selected from herpes simplex virus, adenovirus and poxvirus; more preferably, the herpes simplex virus is preferably herpes simplex virus type I (HSV-1), such as R3616, T-VEC, HF10, G207, NV1020 and OrienX010; the poxvirus is selected from Pexa-Vec (a vaccinia virus), JX-594 (a vaccinia virus), GL-ONC1 and Myxoma; and the adenovirus is selected from Enadenotucirev, DNX-2401, C-REV, NG-348, ProsAtak, CG0070, ADV-TK, EDS01, KH901, H101, H103, VCN-01 and Telomelysin (OBP-301).

Preferably, the ssRNA virus is selected from picornavirus, alphavirus, retrovirus, paramyxovirus and rhabdovirus; preferably, the picornavirus is selected from CAVATAK, PVS-RIPO, CVA21 (an enterovirus) and RIGVIR, the alphavirus is selected from M1, Sindbis AR339 and Semliki Forest virus, the retrovirus is selected from Toca511, the paramyxovirus is selected from MV-NIS and PV701 (a Newcastle disease virus), and the rhabdovirus is selected from VSV-IFNβ, MG1-MAGEA3 and VSV-GP.

Preferably, the dsRNA virus is selected from reovirus; preferably, the reovirus is selected from Pelareorep, Reolysin, vaccinia virus, mumps virus and human immunodeficiency virus (HIV). Preferably, the RNA virus is selected from reovirus, coxsackievirus, poliovirus, porcine Seneca Valley virus, measles virus, Newcastle disease virus, vesicular stomatitis virus and influenza virus.

Preferably, in the pharmaceutical combination as described above, the oncolytic virus expresses an exogenous gene, preferably those of a Bispecific T cell engager (BiTE), an scFv fragment, a cytokine and a chemokine. The BiTE can bind to a molecule that activates T cells such as CD3, and can also bind to an antigen target on the surface of a cancer cell. The scFv targets an immune checkpoint, including CTLA-4, PD-1, TIM-3, LAG3, Siglec15, 4-1BB, GITR, OX40, CD40L, CD28, TIGIT and VISTA. The cytokine and chemokine are, for example, GM-CSF, interleukin-2 (IL-2), interleukin-12 (IL-12), an interferon (IFN), a tumor necrosis factor (TNF), soluble CD80 and CCL3.

### 2.5. Gene therapy drugs

Preferably, in the composition as described above, the gene therapy virus expresses an exogenous gene, which encodes a protein required for a gene-deficient disease selected from acidic α-glucosidase, copper-transporting ATPase 2, α-galactosidase, argininosuccinate synthase, β-glucocerebrosidase, β-hexosaminidase A, Cl protease inhibitors or Cl esterase inhibitors, glucose 6-phosphatase, insulin, glucagon, growth hormone, parathyroid hormone, growth hormone releasing factor, follicle stimulating hormone, luteinizing hormone, human chorionic gonadotropin, vascular endothelial growth factor, angiopoietin, angiostatin, granulocyte colony-stimulating factor, erythropoietin, connective tissue growth factor, basic fibroblast growth factor, acidic fibroblast growth factor, epidermal growth factor, transforming growth factor a, platelet-derived growth factor, insulin growth factors I and II, TGF, bone morphogenetic protein, nerve growth factor, brain-derived neurotrophic factor, neurotrophins NT-3 and NT4/5, ciliary neurotrophic factor, glial cell line-derived neurotrophic factor, neurotrophin, lectin, netrin-1 and netrin-2, hepatocyte growth factor, ephrins, tyrosine hydroxylase, thrombopoietin, interleukins (IL-1 to IL-36, etc.), monocyte chemoattractant protein, leukemia inhibitory factor, granulocyte-macrophage colony-stimulating factor, Fas ligand, tumor necrosis factors a and b, interferon a/b/g, stem cell factor, flk-2/flt3 ligand, IgM, IgA, IgD and IgE, chimeric immunoglobulins, humanized antibodies, single-chain antibodies, T cell receptors, chimeric T cell receptors, single-chain T cell receptors, MHC class I and class II molecules, cystic fibrosis transmembrane regulatory factor, coagulation (clotting) factors (factor XIII, factor IX, factor VIII, factor X, factor VII, factor VIIa, protein C, etc.), retinal pigment epithelium-specific 65 kDa protein, LDL receptor, lipoprotein lipase, ornithine transcarbamylase, β-globulin, α-globulin, spectrin, α-antitrypsin, adenosine deaminase, metal transporter (ATP7A or ATP7), sulfonamidase, enzymes involved in lysosomal storage disease (ARSA), hypoxanthine-guanine phosphoribosyltransferase, b-25 glucocerebrosidase, sphingomyelinase, lysosomal hexosaminidase and branched-chain keto acid dehydrogenase.

Preferably, in the composition as described above, the gene therapy virus carries an exogenous gene, which encodes an inhibitory nucleic acid selected from siRNA, antisense molecules, miRNA, RNAi, ribozymes and shRNA. The inhibitory nucleic acid binds to a polynucleotide repeat disease-related gene, the transcript of which or the polynucleotide of the transcript of which is repeated. The disease-related gene encodes a related protein selected from huntington protein (HTT), androgen receptor on the X chromosome in spinobulbar muscular atrophy, human Ataxin-1/-2/-3/-7, Cav2.1 P/Q voltage-dependent calcium channel (CACNA1A), TATA-binding protein, Ataxin8 opposite strand (ATXN80S), serine/threonine protein phosphatase 2A 55 kDa regulatory subunit B beta isoform in spinocerebellar ataxia (types 1, 2, 3, 6, 7, 8, 12 and 17), FMR1 (Fragile X Mental Retardation 1) in fragile X syndrome, FMR1 (Fragile X Mental Retardation 1) in fragile X-associated tremor/ataxia syndrome, FMR1 (Fragile X Mental Retardation 2) or AF4/FMR2 family member 2 in fragile XE mental retardation, myotonin-protein kinase (MT-PK) in myotonic dystrophy and Frataxin. The disease-related gene is selected from a mutant of superoxide dismutase 1 (SOD1) gene, a gene related to pathogenesis of Parkinson's disease and/or Alzheimer's disease, apolipoprotein B (APOB) gene, PCSK9 gene, HIV infection-related genes (HIVTat, TAR, HIVTAR and CCR5), influenza A virus genome/gene sequences in influenza virus infection, severe acute respiratory syndrome (SARS) coronavirus genome/gene sequences in SARS infection, respiratory syncytial virus genome/gene sequences in respiratory syncytial virus infection, Ebola virus genome/gene sequences in Ebola virus infection, hepatitis B and C virus genome/gene sequences in hepatitis B and C virus infection, herpes simplex virus (HSV) genome/gene sequences in HSV infection, coxsackievirus B3 genome/gene sequences in coxsackievirus B3 infection, silencing of a pathogenic allele of a gene (allele-specific silencing) like torsinA in primary dystonia, pan-class I and HLA-allele specific in transplant, and mutant rhodopsin gene in autosomal dominantly inherited retinitis pigmentosa.

### III. Articles of manufacture

The present invention also provides an article of manufacture, comprising the mutant or protein as described above; and a therapeutic agent selected from a viral vector drug, an antibody, and a polypeptide drug capable of reducing the IgG level in the blood.

The present invention also provides a kit or kit of parts, comprising: 1) a therapeutically effective amount of an agent, including the mutant as described above; and 2) a therapeutically effective amount of a therapeutic agent selected from a viral vector drug, an antibody, and a polypeptide drug capable of reducing the IgG level in the blood; preferably, the viral vector drug is an oncolytic virus or a gene therapy virus. The kit can further comprise 3) a targeted drug or a chemotherapeutic drug or an immune checkpoint blocker. The targeted drug is selected from an epigenetic drug, such as a histone deacetylase inhibitor, an inhibitor targeting the PI3K/Akt/mTOR signaling pathway, such as Tricibine, and a tyrosine kinase inhibitor, such as sunitinib; the chemotherapeutic drug is selected from an immunosuppressant, such as cyclophosphamide, thalidomide and pomalidomide, a proteasome inhibitor, such as bortezomib, a cytotoxic drug, such as gemcitabine and temozolomide, and a cell cycle non-specific drug, such as mitoxantrone; and the immune checkpoint blocker is selected from an anti-CTLA-4 antibody, an anti-PD-1 antibody, an anti-TIM-3 antibody, an anti-LAG3 antibody, an anti-Siglec15 antibody, an anti-4-1BB antibody, an anti-GITR antibody, an anti-OX40 antibody, an anti-CD40L antibody, an anti-CD28 antibody, an anti-TIGIT antibody and an anti-VISTA antibody.

The kit or kit of parts comprises a part A, comprising a therapeutically effective amount of the mutant or protein as described above; and a part B, comprising a therapeutically effective amount of a therapeutic agent selected from a viral vector drug, preferably an oncolytic virus or a gene therapy virus, an antibody, and a polypeptide drug capable of reducing the IgG level in the blood. The kit of parts can further comprise a part C. The part C comprises a targeted drug or a chemotherapeutic drug or an immune checkpoint blocker. The targeted drug is selected from an epigenetic drug, such as a histone deacetylase inhibitor, an inhibitor targeting the PI3K/Akt/mTOR signaling pathway, such as Tricibine, and a tyrosine kinase inhibitor, such as sunitinib; the chemotherapeutic drug is selected from an immunosuppressant, such as cyclophosphamide, thalidomide and pomalidomide, a proteasome inhibitor, such as bortezomib, a cytotoxic drug, such as gemcitabine and temozolomide, and a cell cycle non-specific drug, such as mitoxantrone; and the immune checkpoint blocker is selected from an anti-CTLA-4 antibody, an anti-PD-1 antibody, an anti-TIM-3 antibody, an anti-LAG3 antibody, an anti-Siglec15 antibody, an anti-4-1BB antibody, an anti-GITR antibody, an anti-OX40 antibody, an anti-CD40L antibody, an anti-CD28 antibody, an anti-TIGIT antibody and an anti-VISTA antibody.

The kit can comprise instructions on the administration of the therapeutically effective amount of the mutant or protein as described above and the therapeutically effective amount of the therapeutic agent (e.g., dose information and administration interval information). The therapeutic agent is selected from a viral vector drug, preferably an oncolytic virus or a gene therapy virus, an antibody, and a polypeptide drug capable of reducing the IgG level in the blood.

Well-established expression systems can be used to prepare viral vector drugs. Some examples of methods include the use of mammalian cell expression systems to produce viral particles, such as the use of HEK293 cells to produce adenovirus viral vector drugs (Freedman Joshua D, Duffy Margaret R, Lei-Rossmann Janet et al., An Oncolytic Virus Expressing a T-cell Engager Simultaneously Targets Cancer and Immunosuppressive Stromal Cells. [J].Cancer Res., 2018, 78: 6852-6865).

The pharmaceutical carrier can be liquid, and the pharmaceutical composition can be in the form of a solution. Liquid carriers are used to prepare solutions, suspensions, emulsions, syrups, elixirs and pressurized compositions. The active ingredients can be dissolved or suspended in a pharmaceutically acceptable liquid carrier, such as water, an organic solvent, a mixture of the two, or a pharmaceutically acceptable oil or fat.

The pharmaceutical composition for parenteral administration is sterile, substantially isotonic, and pyrogen-free, and is prepared in accordance with the GMP of the FDA or a similar agency. The viral vector drug can be administered as an injectable dosage form of a solution or suspension thereof, wherein the substance is in physiologically acceptable diluent and pharmaceutical carrier (which can be a sterile liquid, such as water, oil, saline, glycerol or ethanol). In addition, an auxiliary substance such as a wetting agent or an emulsifier, a surfactant and a pH buffering substance can be present in the composition. Other components of the pharmaceutical composition include those of petroleum, animal, plant or synthetic origin, such as peanut oil, soybean oil and mineral oil. In general, diols such as propylene glycol or polyethylene glycol are preferred liquid carriers, especially for injectable solutions. The viral vector drug can be administered in the form of a depot injection or an implanted preparation, which can be formulated to allow sustained release of the active ingredient. Typically, the composition is prepared as an injectable preparation, i.e., a liquid solution or suspension, or can be prepared as a solid form suitable for dissolution or suspension in a liquid carrier prior to injection.

Unless otherwise defined, all technical and scientific terms used herein have the same meanings as commonly understood by those of ordinary skill in the art to which the present invention belongs. While any methods, devices and materials similar or equivalent to those described herein can be used in the practice or testing of the present invention, preferred methods, devices and materials are now described.

The term "nucleotide" or "polynucleotide" means a single-stranded or doublestranded deoxyribonucleotide, deoxyribonucleoside, ribonucleoside or ribonucleotide and polymers thereof. Unless specifically limited, the terms cover nucleic acids containing known analogs of natural nucleotides that have binding properties similar to reference nucleic acids and are metabolized in a manner similar to naturally occurring nucleotides. Unless otherwise limited specifically, the terms also mean oligonucleotide analogs, including PNAs (peptide nucleic acids), DNA analogs used in antisense techniques (phosphorothioate, phosphoramidate, etc.), etc. Unless otherwise specified, a particular nucleic acid sequence also implicitly encompasses its conserved modified variants (including, but not limited to, degenerate codon substitutions) and complementary sequences as well as explicitly specified sequences. In particular, degenerate codon substitution can be achieved by generating a sequence in which the 3rd position of one or more selected (or all) codons is substituted with a mixed base and/or deoxyinosine residue (Batzer et al., Nucleic Acid Res. 19: 5081 (1991); Ohtsuka et al., J. Biol. Chem. 260: 2605-2608 (1985); and Cassol *et al.,* (1992); Rossolini et al., Mol Cell. Probes 8: 91-98 (1994)).

The terms "polypeptide" and "protein" are used interchangeably herein to mean polymers of amino acid residues. In other words, the description of a polypeptide is equally applicable to the description of a peptide and a protein, and vice versa. The terms apply to naturally occurring amino acid polymers, and amino acid polymers in which one or more amino acid residues are non-naturally encoded amino acids. As used herein, the terms encompass amino acid chains of any length, including full-length proteins (i.e., antigens), in which amino acid residues are linked via covalent peptide bonds.

The term "host cell" means a cell comprising the nucleotide of the present invention, regardless of the method used for insertion to produce a recombinant host cell, such as direct uptake, transduction, pairing, or other methods known in the art. An exogenous polynucleotide can exist as a non-integrated vector such as a plasmid, or can be integrated into the host's genome. The host cell can be a prokaryotic cell or a eukaryotic cell.

The term "transformation" means a process by which a heterologous DNA sequence is introduced into a host cell or organism.

The term "expression" means the transcription and/or translation of an endogenous gene or a transgene in a cell.

The positive and progressive effect of the present invention is as follows: The present invention provides a mutant of an immunoglobulin-degrading enzyme, which has higher activity and/or thermal stability than the activity and/or thermal stability of the wild-type IdeE, and higher activity than that of IdeS and IdeZ (which is more effective than IdeS and IdeZ in cleaving human IgG, and the activity of which is nearly twice that of IdeS, and more than 4 times that of IdeZ).

### Examples

The present invention will be further described by way of examples below, but the present invention is not limited to the scope of the described examples. The experimental methods in the following examples that do not indicate the specific conditions are selected according to conventional methods and conditions or according to the product's instructions.

### Example 1. Design and expression of mutant library

A mutant library of the wild-type IdeE protein sequence was designed and constructed, and forty mutants were obtained through screening.

The polynucleotide sequence encoding the wild-type IdeE protein sequence (SEQ ID NO: 2) was synthesized by codon optimization. An N-terminal signal peptide sequence and a C-terminal 6× histidine tag were added. A sequence was synthesized, and then inserted into the pET32a expression vector. A recombinant plasmid used to express the wild-type IdeE was obtained after being verified to be correct by sequencing. Based on the expression plasmid of the wild-type IdeE, degenerate primers required for a mutant library were designed. The original wild-type sequence was amplified, and the amplified sequence was inserted into the vector to obtain a mutant library recombinant plasmid. The wild-type and mutant library recombinant plasmids were electrotransformed into *Escherichia coli* BL21 Star (DE3), and the cells were plated on an LB agarose plate containing 100 ug/ml of ampicillin. The plate was cultured at 37°C overnight until colonies grow out. Single colonies were picked and inoculated to 200 ul of LB medium containing 100 ug/ml of ampicillin, and cultured at 37°C and 250 rpm overnight. The overnight cultures were inoculated to 1 ml of LB medium containing 100 ug/ml of ampicillin, and cultured at 37°C for 4 h. Then, 0.1 mM of IPTG was added, and the mixtures were further cultured at 30°C overnight. Supernatants were harvested by centrifugation from the overnight cultures. SDS-PAGE was used to evaluate the concentrations of the mutant proteins in the mutant expression supernatants.

### Example 2. Assessment of human IgG1-cleaving activity of mutants

In order to evaluate the activity of cleaving human IgG1 of each mutant, an ELSIA-based activity assay method was established. The principle of the assay was as follows: An ELSIA plate was coated with a human IgG1-specific antigen, and then the supernatant samples containing an equivalent concentration of mutant proteins were incubated with human IgG1 in the wells. A human IgG1 detection antibody specific to the antibody Fc portion was used to measure the amount of intact or partially-cleaved human IgG1 bound to the well. In the case that the concentrations of the mutant proteins in the given supernatants in the wells were the same, the higher the activity of cleaving human IgG1 of the mutant protein was, the less intact anti-human IgG1 antibody bound to the well, thus obtaining a lower signal. An IgG1 standard curve can be generated based on the relationship between different concentrations of IgG1 and the corresponding detection signals. According to the standard curve, the amount of intact or partially-cleaved IgG1 was calculated, and then the amount of fully-cleaved IgG1 was calculated. The activity of the mutant was evaluated based on the proportion of fully-cleaved IgG1 to the initial IgG1.

In order that the concentrations of the mutant proteins in the supernatants harvested in Example 1 were equivalent, SDS-PAGE was performed with the same loading amount. The optical density value of a protein band of interest in the electrophoretogram was analyzed using Quantity One. In the case that the loading amounts were the same, the higher the optical density value of the protein band of interest in the pattern was, the higher the concentration was. Using the IdeE supernatant as a control, the other mutant supernatants were concentrated or diluted, so that the optical density values of the mutant protein bands were all substantially the same as that of the IdeE control.

After adjusting the protein concentrations in the supernatants to an equivalent level, ELISA was carried out as follows: An ELISA plate was coated with 2 ug/ml of human IgG1 (trastuzumab)-specific antigen (Art.No. QRE-104, RUIAN BIOTECHNOLOGY) at 2-8°C overnight, and then washed with PBST (PBS + 0.05% Tween20). The washed ELISA plate was blocked with 2% BSA (formulated in PBS) at 37°C for 2 h, and then washed with PBST.

Generation of standard curve: 200 ng/ml of trastuzumab solution was subjected to gradient dilution with reaction buffer (10 mM PB, 10 mM NaCl, pH 6.5) at a ratio of 1 : 2 to 3.125 ng/ml. 100 ul of diluents with different concentrations of trastuzumab were added into the wells of the ELISA plate for the generation of a standard curve of substrate (trastuzumab).

Cleavage reaction: After adjusting the protein concentration, the supernatants were diluted 5x with reaction buffer (10 mM PB, 10 mM NaCl, pH 6.5). 50 ul of 100 ng/ml trastuzumab diluent and 50 ul of diluted supernatants were added into the wells of the ELISA plate.

The ELISA plate was incubated with shaking at 37°C for 1 h, and washed with PBST. Then, 40 ng/ml of Goat anti-Human IgG Fc Cross-Adsorbed Secondary Antibody-HRP (Art.No. 31413, Thermo) was added into the wells of the plate, and the plate was incubated with shaking at 37°C for 1 h and washed with PBST. Then, TMB was added as HRP chromogenic substrate. The plate was incubated for 15 min, and then the reaction was terminated with 2N H₂SO₄. The absorbance at 450 nm was detected by a microplate reader. According to the substrate standard curve, the concentrations of intact or partially-cleaved trastuzumab in different test wells were calculated, and then the proportions of fully-cleaved trastuzumab to the initial trastuzumab were calculated, so as to evaluate the activity of different mutants.

The multiple relationship between the activity of each mutant relative to that of the wild-type IdeE is shown in Table 1. The forty mutants obtained through screening in Example 1 all had higher activity than that of the wild-type IdeE, of which fifteen mutants were twice or more as active as the wild-type IdeE.

**Table 1. Multiple relationships between the activity of the mutants relative to that of the wild-type IdeE measured by ELISA**

| Mutant | Fold of activity relative to wild-type | SEQ ID NO: | Mutant | Fold of activity relative to wild-type | SEQ ID NO: |
|---|---|---|---|---|---|
| T8D | 2.21 | 3 | T24S | 2.18 | 11 |
| T8E | 2.09 | 4 | A59I | 2.31 | 12 |
| T8W | 2.58 | 5 | A59L | 2.41 | 13 |
| T8Y | 2.30 | 6 | A59V | 2.68 | 14 |
| A10K | 1.99 | 7 | E97D | 2.00 | 15 |
| A10R | 2.20 | 8 | R280H | 2.30 | 16 |
| T24A | 2.23 | 9 | R280K | 2.20 | 17 |
| T24G | 2.09 | 10 | - | - | - |

### Example 3. Assessment of thermal stability of mutants

Twelve mutants were selected from the fifteen mutants shown in Table 1 which were twice or more as active as the wild-type IdeE for the detection of their thermal stability. Detection method: The activity detection method was as follows:
The supernatant of the wild-type or each mutant was divided into two parts, and the two parts were placed at 4°C and 50°C for 1 h, respectively. Subsequently, the activity of the wild-type or each mutant was detected according to the ELISA method in Example 2. The residual activity percentage (%) of the wild-type or each mutant after being placed at 50°C for 1 h was calculated based on the activity after being placed at 50°C/the activity after being placed at 4°C, so as to compare the thermal stability of the wild-type and each mutant.

The multiple relationships between the thermal stability of the mutants relative to that of the wild-type IdeE are shown in Table 2. Table 2 shows that the twelve mutants all had higher thermal stability than that of the wild-type, of which seven mutants had thermal stability more than 3 times that of the wild-type.

**Table 2. Multiple relationships between the thermal stability of the mutants relative to that of the wild-type IdeE**

| Mutant | Fold of thermal stability relative to wild-type | SEQ ID NO: | Mutant | Fold of thermal stability relative to wild-type | SEQ ID NO: |
|---|---|---|---|---|---|
| T8D | 4.14 | 3 | A59V | 5.20 | 14 |
| T8W | 4.43 | 5 | E97D | 4.27 | 15 |
| T24A | 4.50 | 9 | R280H | 4.36 | 16 |
| A59L | 3.83 | 13 | - | - | - |

### Example 4. Comparison of human IgG1-cleaving activity of single-point mutants

The activity of cleaving human IgG1 of the seven single-point mutants as shown in Tables 1 and 2, T8D, T8W, T24A, A59L, A59V, E97D and R280H, was detected, "whose activity was more than twice that of the wild-type IdeE, and whose thermal stability was more than 3 times that of the wild-type IdeE".

### 1. Expression and purification of mutants

From each of the plates transformed with the above-mentioned five single-point mutants in Example 1, one single colony was picked, inoculated to 3 ml of LB medium containing 100 ug/ml of ampicillin, and cultured at 37°C and 250 rpm overnight. The overnight cultures were inoculated to 50 ml of LB medium containing 100 ug/ml of ampicillin, and cultured at 37°C until OD600 reached 0.4-0.6. Then, 0.1 mM of IPTG was added, and the mixtures were further cultured at 30°C overnight. Supernatants were harvested by centrifugation from the overnight cultures. The supernatants were further purified with IDA-Ni agarose magnetic beads, and the purified and eluted proteins were buffer exchanged into PBS buffer system with an ultrafiltration centrifuge tube. SDS-PAGE was used to evaluate the purity of the purified mutant proteins. OD280 was detected, and the concentration of the purified mutant proteins was calculated according to the extinction coefficient.

### 2. Comparison of human IgG1-cleaving activity of mutants

The activity of cleaving human IgG1 of different mutants relative to the wild-type IdeE was further evaluated via the cleavage products resulting from cleavage of human IgG1 by each mutant at different concentrations displayed on SDS-PAGE. The purified mutants and the wild-type IdeE were diluted to 0.002 mg/mL and 0.001 mg/mL, respectively. 50 ul of mutants or wild-type IdeE at different concentrations was each added to 50 ul of reaction system containing 2 mg/ml of trastuzumab to initiate the cleavage reaction, and the reaction systems were placed at 37°C for 30 min. The samples were mixed with an equal volume of 2× SDS loading buffer, and then placed in water bath at 75°C for 5 min. The cleavage products were detected by SDS-PAGE.

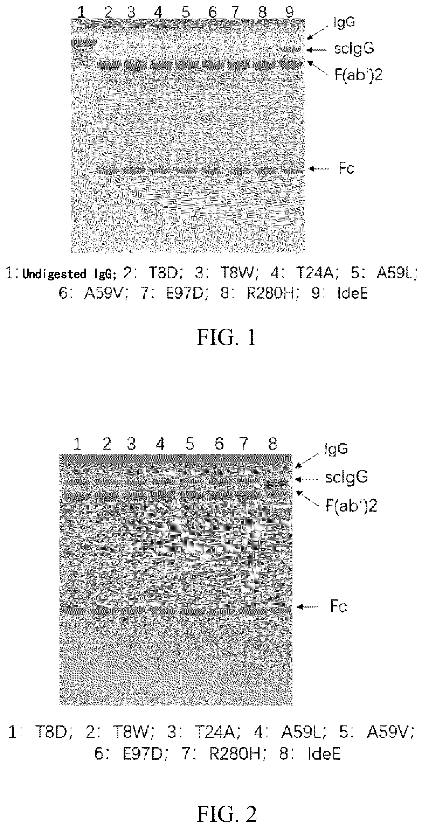

Figure 1 shows the electrophoretogram of cleavage products resulting from cleavage of human IgG1 by the seven single-point mutants and the wild-type IdeE (enzyme : substrate = 1 : 1000). Figure 2 shows the electrophoretogram of cleavage products resulting from cleavage of human IgG1 by the seven single-point mutants and the wild-type IdeE (enzyme : substrate = 1 : 2000). The seven single-point mutants at the concentration of 0.001 mg/ml all cleaved IgG1 with an efficiency not worse than that of 0.002 mg/ml of wild-type IdeE, indicating that the activity of cleaving human IgG1 of the seven single-point mutants is not less than twice that of the wild-type IdeE.

### Example 5. Comparison of human IgG1-cleaving activity and thermal stability of N-terminal truncated mutants

On the basis of the wild-type IdeE, the first 15 amino acids (D1-V15), the first 16 amino acids (D1-P16), the first 17 amino acids (D1-H17), the first 18 amino acids (D1-Q18) and the first 19 amino acids (D1-I19) at its N-terminus were respectively deleted, so as to construct five N-terminal truncated mutants (see Table 3).

**Table 3. Sequence design of truncated mutants**

| Mutant | Modification relative to wild-type or mutant sequence | SEQ ID NO: |
|---|---|---|
| WT_del15 | Deletion of the first 15 amino acids in SEQ ID NO: 2 | 18 |
| WT_del16 | Deletion of the first 16 amino acids in SEQ ID NO: 2 | 19 |
| WT_del17 | Deletion of the first 17 amino acids in SEQ ID NO: 2 | 20 |
| WT_del18 | Deletion of the first 18 amino acids in SEQ ID NO: 2 | 21 |
| WT_del19 | Deletion of the first 19 amino acids in SEQ ID NO: 2 | 22 |

### 1. Expression and purification of mutants

According to the method in Example 1, the polynucleotide sequences of the mutants in Table 3 were synthesized, and the mutant expression recombinant plasmids were constructed to transform *Escherichia coli* BL21 Star (DE3). The mutant purified proteins were prepared according to the method in Example 4.

### 2. Comparison of human IgG1-cleaving activity of mutants

The purified mutants and the wild-type IdeE were diluted to 0.002 mg/mL, respectively. 50 ul of diluted mutants or wild-type IdeE was each added to 50 ul of reaction system containing 2 mg/ml of trastuzumab to initiate the cleavage reaction, and the reaction systems were placed at 37°C for 30 min. The samples were mixed with an equal volume of 2× SDS loading buffer, and then placed in water bath at 75°C for 5 min. The cleavage products were detected by SDS-PAGE.

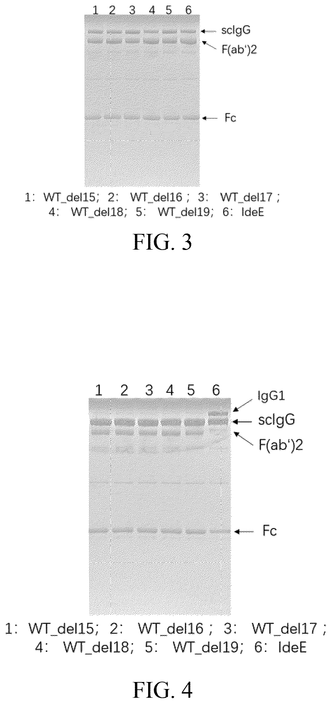

Figure 3 shows the electrophoretogram of cleavage products resulting from cleavage of human IgG1 by the five truncated mutants (enzyme : substrate = 1 : 1000). The cleavage activity of all of the five truncated mutants was not significantly different from that of the wild-type IdeE.

### 3. Comparison of thermal stability of mutants

The purified mutants and the wild-type IdeE were respectively diluted to 0.1 mg/ml, placed at 50°C for 1 h, and then further diluted to 0.002 mg/mL. 50 ul of diluted mutants or wild-type IdeE was each added to 50 ul of reaction system containing 2 mg/ml of trastuzumab to initiate the cleavage reaction, and the reaction systems were placed at 37°C for 30 min. The samples were mixed with an equal volume of 2× SDS loading buffer, and then placed in water bath at 75°C for 5 min. The cleavage products were detected by SDS-PAGE.

Figure 4 shows the electrophoretogram of cleavage products resulting from cleavage of human IgG1 by the five truncated mutants and the wild-type IdeE after being placed at 50°C for 1 h (enzyme : substrate = 1 : 1000). The residual activity of the five truncated mutants after heat treatment at 50°C was significantly higher than that of the wild-type, indicating that the thermal stability of all of the five truncated mutants is significantly improved compared with that of the wild-type.

### Example 6. Comparison of human IgG1-cleaving activity of C-terminal truncated mutants

On the basis of the wild-type IdeE, the last 5 amino acids (W311-S315) and the last 10 amino acids (S306-S315) at its C-terminus were respectively deleted, so as to construct two C-terminal truncated mutants (see Table 4).

**Table 4. Sequence design of truncated mutants**

| Mutant | Modification relative to wild-type or mutant sequence | SEQ ID NO: |
|---|---|---|
| WT_delC5 | Deletion of the last 5 amino acids in SEQ ID NO: 2 | 23 |
| WT_delC10 | Deletion of the last 10 amino acids in SEQ ID NO: 2 | 24 |

### 1. Expression and purification of mutants

According to the method in Example 1, the polynucleotide sequences of the mutants in Table 4 were synthesized, and the mutant expression recombinant plasmids were constructed to transform *Escherichia coli* BL21 Star (DE3). The mutant purified proteins were prepared according to the method in Example 4.

### 2. Comparison of human IgG1-cleaving activity of mutants

The purified mutants and the wild-type IdeE were diluted to 0.002 mg/mL, respectively. 50 ul of diluted mutants or wild-type IdeE was each added to 50 ul of reaction system containing 2 mg/ml of trastuzumab to initiate the cleavage reaction, and the reaction systems were placed at 37°C for 30 min. The samples were mixed with an equal volume of 2× SDS loading buffer, and then placed in water bath at 75°C for 5 min. The cleavage products were detected by SDS-PAGE.

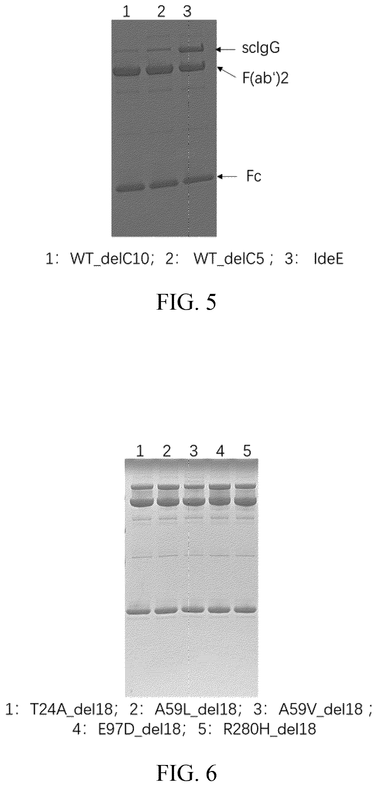

Figure 5 shows the electrophoretogram of cleavage products resulting from cleavage of human IgG1 by the two C-terminal truncated mutants (enzyme : substrate = 1 : 1000). The cleavage activity of both of the two truncated mutants was more than twice that of the wild-type IdeE.

### Example 7. Comparison of human IgG1-cleaving activity and thermal stability of combinatorial mutants

On the basis of the five single-point mutants, T24A, A59L, A59V, E97D and R280H, the first 18 amino acids (D1-Q18) were respectively deleted, so as to construct five combinatorial mutants (see Table 5).

**Table 5. Sequence design of combinatorial mutants**

| Mutant | Modification relative to wild-type or mutant sequence | SEQ ID NO: |
|---|---|---|
| T24A_del18 | Deletion of the first 18 amino acids in SEQ ID NO: 9 | 25 |
| A59L_del18 | Deletion of the first 18 amino acids in SEQ ID NO: 13 | 26 |
| A59V_del18 | Deletion of the first 18 amino acids in SEQ ID NO: 14 | 27 |
| E97D_del18 | Deletion of the first 18 amino acids in SEQ ID NO: 15 | 28 |
| R280H_del18 | Deletion of the first 18 amino acids in SEQ ID NO: 16 | 29 |

### 1. Expression and purification of mutants

According to the method in Example 1, the polynucleotide sequences of the mutants in Table 5 were synthesized, and the mutant expression recombinant plasmids were constructed to transform *Escherichia coli* BL21 Star (DE3). The mutant purified proteins were prepared according to the method in Example 4.

### 2. Comparison of human IgG1-cleaving activity of mutants

The purified mutants were diluted to 0.001 mg/mL, respectively. 50 ul of diluted mutants or wild-type IdeE was each added to 50 ul of reaction system containing 2 mg/ml of trastuzumab to initiate the cleavage reaction, and the reaction systems were placed at 37°C for 30 min. The samples were mixed with an equal volume of 2× SDS loading buffer, and then placed in water bath at 75°C for 5 min. The cleavage products were detected by SDS-PAGE.

Figure 6 shows the electrophoretogram of cleavage products resulting from cleavage of human IgG1 by the five combinatorial mutants (enzyme : substrate = 1 : 2000). Comparing the cleavage in Figure 6 to that in Figure 2, there was no significant difference in cleavage activity between the five truncated mutants and the five single-point combinatorial mutants, indicating that the activity of cleaving human IgG1 of the combinatorial mutants is also not less than twice that of the wild-type IdeE.

### 3. Comparison of thermal stability of mutants

The purified mutants were respectively diluted to 0.1 mg/ml, placed at 50°C for 1 h, and then further diluted to 0.001 mg/mL. 50 ul of diluted mutants or wild-type IdeE was each added to 50 ul of reaction system containing 2 mg/ml of trastuzumab to initiate the cleavage reaction, and the reaction systems were placed at 37°C for 30 min. The samples were mixed with an equal volume of 2× SDS loading buffer, and then placed in water bath at 75°C for 5 min. The cleavage products were detected by SDS-PAGE.

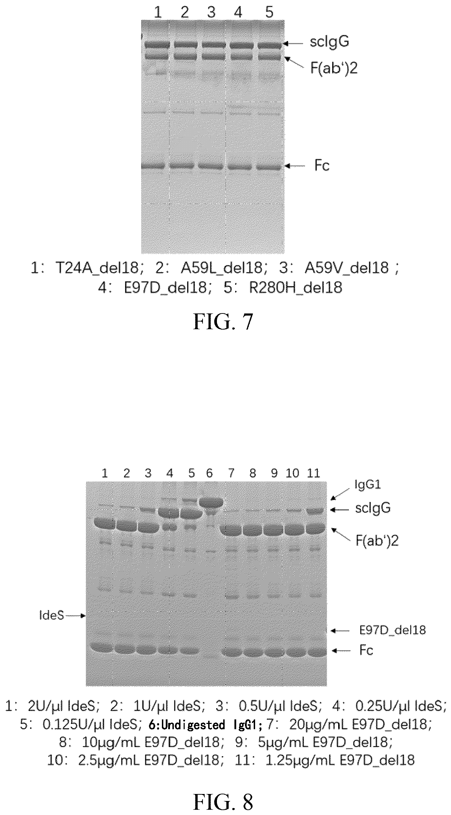

Figure 7 shows the electrophoretogram of cleavage products resulting from cleavage of human IgG1 by the five combinatorial mutants after being placed at 50°C for 1 h (enzyme : substrate = 1 : 2000). Comparing the cleavage in Figure 8 to that in Figure 7, the activity of the five combinatorial mutants after heat treatment at 50°C only decreased slightly, indicating that the thermal stability of all of the five combinatorial mutants is also significantly improved compared with that of the wild-type.

### Example 8. Comparison of activity of mutant E97D del18 to that of IdeS and IdeZ

The purified E97D_del18 mutant in Example 7 was sequentially diluted to 20 µg/mL, 10 µg/mL, 5 µg/mL, 2.5 µg/mL and 1.25 µg/ml. IdeS (FabRICATOR^{®}, Art.No. A0-FRI-020, Genovis) was respectively diluted to 2 U/µl, 1 U/µl, 0.5 U/µl, 0.25 U/µl and 0.125 U/µl according to its label. IdeZ (FabRICATOR-Z^{®}, Art.No. A0-FRZ-020, Genovis) was diluted to 0.4 U/µl, 0.2 U/µl, 0.1 U/µl, 0.05 U/µl and 0.025 U/µl, respectively. 50 µl of mutants, IdeS or IdeZ at different concentrations was each added to 50 µl of reaction system containing 2 mg/ml of trastuzumab to initiate the cleavage reaction, and the reaction systems were placed at 37°C for 30 min. The samples were mixed with an equal volume of 2× SDS loading buffer, and then placed in water bath at 75°C for 5 min. The cleavage products were detected by SDS-PAGE.

Figure 8 shows the electrophoretogram of cleavage products resulting from cleavage of human IgG1 by mutant E97D_del18 and IdeS at different concentrations. From the enzyme protein bands on the electrophoretogram, it can be judged that the concentration of enzyme IdeS in lane 1 was between those of mutant enzyme E97D_del18 in lanes 7 and 8, thus the concentration of enzyme IdeS in lane 3 was deduced to be between those of mutant enzyme E97D_del18 in lanes 9 and 10, and the enzymatic digestion of IgG1 in lane 3 was between those in lanes 10 and 11, and therefore it can be inferred that the activity of cleaving human IgG1 of mutant E97D_del18 is nearly twice that of IdeS.

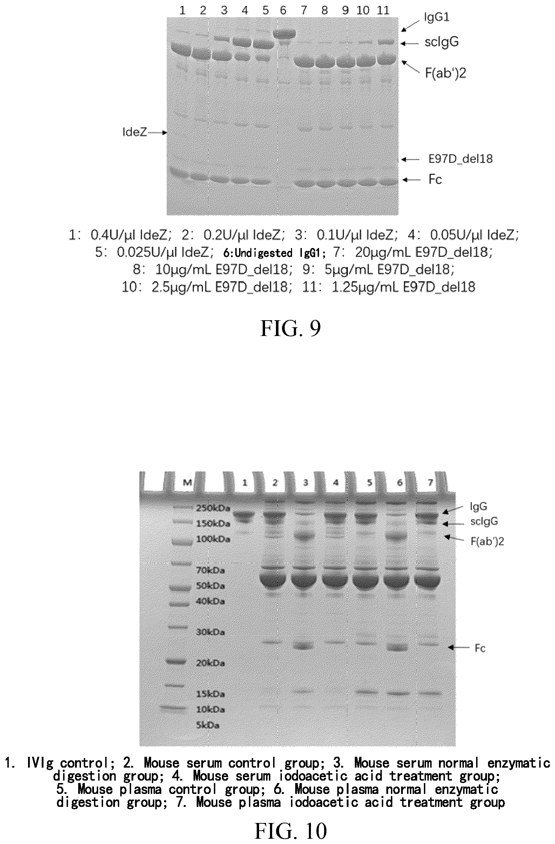

Figure 9 shows the electrophoretogram of cleavage products resulting from cleavage of human IgG1 by mutant E97D_del18 and IdeZ at different concentrations. From the enzyme protein bands on the electrophoretogram, it can be judged that the concentration of enzyme IdeZ in lane 1 was higher than that of mutant enzyme E97D_del18 in lane 7, thus the concentration of enzyme IdeZ in lane 3 was deduced to be higher than that of mutant enzyme E97D_del18 in lane 9, that is, 4 times higher than that of mutant enzyme E97D_del18 in lane 11, and the enzymatic digestion of IgG1 in lane 3 was close to that in lane 11, and therefore it can be inferred that the activity of cleaving human IgG1 of mutant E97D_del18 is 4 times higher than that of IdeZ.

### Example 9. In vitro detection of human IgG1-cleaving activity of mutant E97D del18

The activity of cleaving human IgG1 *in vitro* of mutant E97D_del18 was evaluated by detecting the amount of intact or single-cleaved IVIg in the sera or plasmas of mice treated with mutant E97D_del18 and human IVIg. According to Table 6, mouse serum or plasma enzymatic digestion systems were formulated for different groups.

**Table 6. Mouse serum or plasma enzymatic digestion systems**

| Group name | Volume of PBS (µl) | Volume of serum or plasma (µl) | Concentration of IVIg in the system (mg/ml) | Concentration of E97D_del18 in the system (mg/ml) | Concentration of iodoacetic acid in the system (mM) |
|---|---|---|---|---|---|
| IVIg control | 100 | / | 10 | / | / |
| Mouse serum control group | / | 100 | 10 | / | / |
| Mouse serum normal enzymatic digestion group | / | 100 | 10 | 0.05 | / |
| Mouse serum iodoacetic acid treatment group | / | 100 | 10 | 0.05 | 2 |
| Mouse plasma control group | / | 100 | 10 | / | / |
| Mouse plasma normal enzymatic digestion group | / | 100 | 10 | 0.05 | / |
| Mouse plasma iodoacetic acid treatment group | / | 100 | 10 | 0.05 | 2 |

The effect of iodoacetic acid in the iodoacetic acid treatment groups was to inhibit the activity of the IgG-degrading enzyme.

The systems were placed at 37°C for 30 min. 20 µl of samples were mixed with an equal volume of 2× SDS non-reducing loading buffer, and then placed in water bath at 75°C for 5 min. The cleavage products were detected by SDS-PAGE.

Figure 10 shows the electrophoretogram of cleavage products resulting from cleavage of human IVIg by mutant E97D_del18 in the sera and plasmas of mice. The results showed that E97D_del18 can effectively cleave human IVIg in both the sera and plasmas of mice.

Whether mutant E97D_del18 has the activity of cleaving human IgG1 *in vitro* was evaluated by detecting the sera of mice or humans treated with mutant E97D_del18. According to Table 7, mouse or human serum enzymatic digestion systems were formulated for different groups.

**Table 7. Mouse or human serum enzymatic digestion systems**

| Group name | Volume of mouse serum (µl) | Volume of normal human serum (µl) | Concentration of E97D_del18 in the system (mg/ml) |
|---|---|---|---|
| Mouse serum control group | 100 | / | / |
| Mouse serum enzymatic digestion group | 100 | / | / |
| Human serum control group | / | 100 | 0.02 |
| Human serum enzymatic digestion group | / | 100 | 0.02 |

The systems were placed at 37°C for 24 h. 20 µl of samples were mixed with an equal volume of 2× SDS reducing loading buffer, then diluted 20x with 1× SDS reducing loading buffer, and placed in water bath at 75°C for 5 min. The cleavage products were detected by SDS-PAGE.

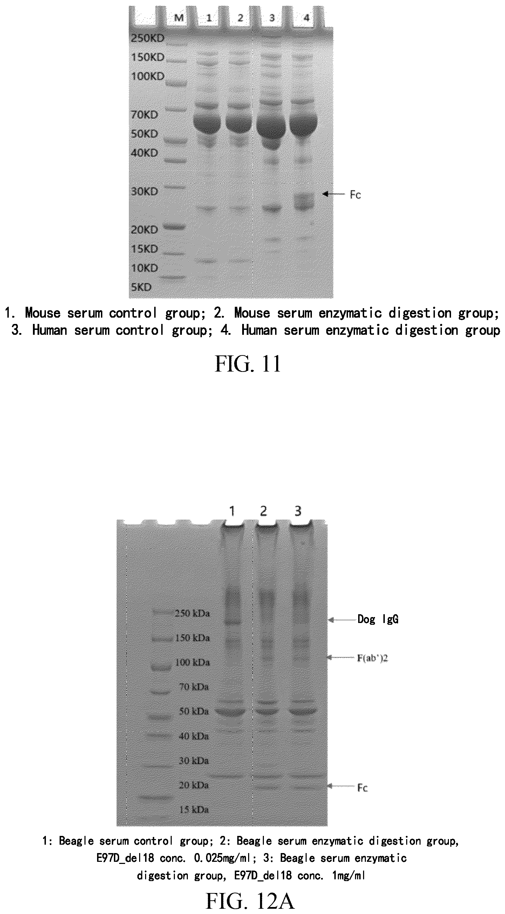

Figure 11 shows the electrophoretogram of cleavage products produced by mutant E97D_del18 in the sera of mice and humans. The results showed that a significantly visible 25 kD Fc fragment was produced in the sera of humans through E97D_del18 cleavage, which was invisible in the sera of mice, indicating that E97D_del18 can effectively and specifically cleave IgG1 in the sera of humans, and has very low or no activity of cleaving IgG1 in the sera of mice.

### Example 10. Cleavage of immunoglobulins of different species by mutant E97D del18

The activity of cleaving serum immunoglobulins of different species of animals *in vitro* of mutant E97D_del18 was evaluated by detecting the amount of intact or single-cleaved IgG in the sera or plasmas of different species of animals added with mutant E97D_del18. According to Tables 8 and 9, serum or antibody enzymatic digestion systems were formulated for different species.

**Table 8. Beagle serum and antibody (of different species) enzymatic digestion systems**

| Group name | Concentration of immunoglobulin in the system (mg/ml) | Concentration of E97D_del18 in the system (mg/ml) |
|---|---|---|
| Beagle serum control group | 10 | / |
| Beagle serum enzymatic digestion group | 10 | 0.025/1 |
| Rabbit polyclonal IgG antibody 1 control group | 1 | / |
| Rabbit polyclonal IgG antibody 1 control group | 1 | 0.005 |
| Rabbit polyclonal IgG antibody 2 control group | 1 | / |
| Rabbit polyclonal IgG antibody 2 control group | 1 | 0.005 |
| Mouse monoclonal antibody IgG1 control group | 1 | 0.005 |
| Mouse monoclonal antibody IgG1 enzymatic digestion group | 1 | 0.005 |
| Mouse monoclonal antibody IgG2a control group | 1 | 0.005 |
| Mouse monoclonal antibody IgG2a enzymatic digestion group | 1 | 0.005 |

The systems were placed at 37°C for 1 h. The enzymatic digestion products were detected by SDS-PAGE.

**Table 9. Serum enzymatic digestion systems of different species**

| Group name | Concentration of immunoglobulin in the system (mg/ml) | Concentration of E97D_dell8 in the system (mg/ml) |
|---|---|---|
| SD rat serum control group | 5 | / |
| SD rat serum enzymatic digestion group | 5 | 0.025/0.5 |
| ICR mouse serum control group | 5 | / |
| ICR mouse serum enzymatic digestion group | 5 | 0.025/0.5 |
| New Zealand rabbit serum control group | 5 | / |
| New Zealand rabbit serum enzymatic digestion group | 5 | 0.025/0.5 |
| Beagle serum control group | 5 | / |
| Beagle serum enzymatic digestion group | 5 | 0.025/0.5 |
| Cynomolgus monkey serum control group | 5 | / |
| Cynomolgus monkey serum enzymatic digestion group | 5 | 0.025/0.5 |
| Bama mini-pig serum control group | 5 | / |
| Bama mini-pig serum enzymatic digestion group | 5 | 0.025/0.5 |

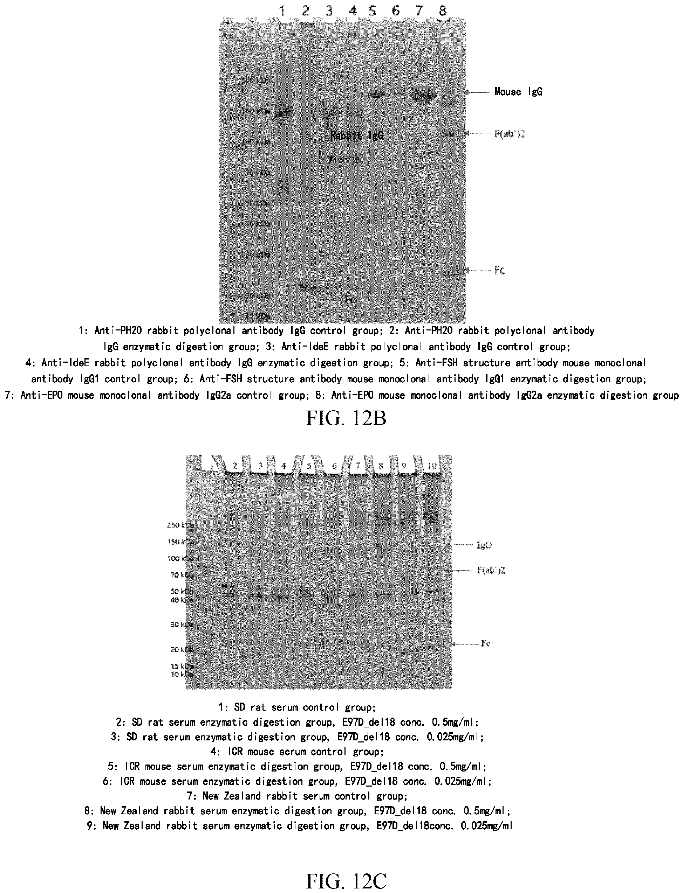

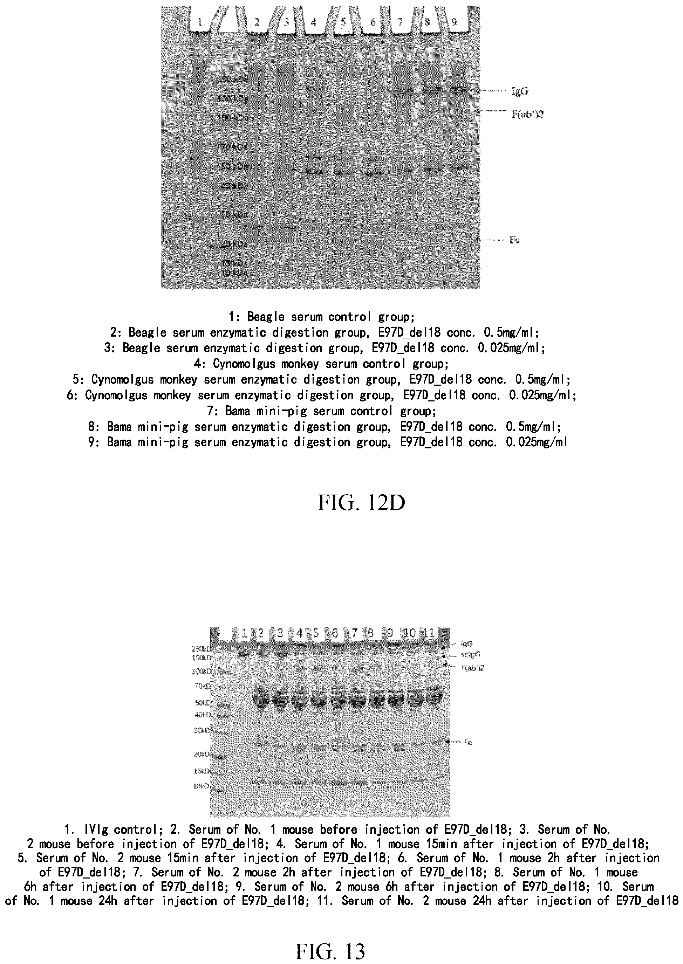

Figures 12A-12D show the cleavage activity of mutant E97D_del18 on the sera and antibodies of different species. The results showed that E97D_del18 can effectively cleave dog IgG, rabbit IgG and mouse IgG2a, but can not cleave mouse IgG1. E97D_del18 can effectively cleave IgG in the sera of rabbits, dogs and monkeys, of which IgG in the sera of rabbits was cleaved most effectively, IgG in the sera of pigs was cleaved more effectively, and IgG in the sera of rats and mice was nearly uncleaved.

### Example 11. Low pre-existing antibodies against mutant E97D del18 in humans

The assay was based on the competition between mutant E97D _del 18 and IdeS for binding to anti-E97D_del18/IdeS antibodies. Pre-incubation of test enzymes and human serum would enable anti-E97D_del18/IdeS antibodies to bind to mutant E97D_del18 and IdeS.

A well plate was coated with mutant E97D_del18 and IdeS overnight, then washed with PBST, and blocked in 2% BSA blocking solution for 1 h. A mixed plate was prepared with the mutant to be tested and IdeS diluted stepwise and human serum. The mixed plate was incubated with shaking at room temperature for 1 h, and washed with PBST. Then, biotin-labeled E97D_del18 mutant and IdeS were added, followed by SA-HRP. The plate was developed with TMB, and read. Parallel comparison obtained the presence of pre-existing antibodies against E97D_del18 and IdeS in about eighty human blood samples.

The results are shown in Table 10, showing that the proportion of pre-existing antibodies against IdeS in normal human serum was as high as about 90%, while for mutant E97D_del18, it was only about 20%. The pre-existing antibodies against mutant E97D_del18 *in vivo* were significantly less than those against IdeS, demonstrating that mutant E97D_del18 has lower immunogenicity, and is more conducive to *in vivo* administration.

**Table 10. Comparison of pre-existing antibodies against mutant E97D_del18 and IdeS in human blood samples**

| | Mutant E97D_del18 | IdeS |
|---|---|---|
| Total number of human blood samples (cases) | 76 | 76 |
| Proportion of samples positive for pre-existing antibodies (%) | 18.4 | 89.5 |

### Example 12. In vivo detection of human IgG1-cleaving activity of mutant E97D del18

Under sterile conditions, two mice were injected with human IVIg (an intravenous human immunoglobulin) at a dose of 1 g/kg intraperitoneally (two mice were parallel experiments, numbered No. 1 and No. 2). 24 h after injecting human IVIg, the mice were injected with the IgG-degrading enzyme (E97D_del18) at a dose of 5 mg/kg intravenously. 0 h, 15 min, 2 h, 6 h and 24 h after injecting E97D_del18, blood was collected from the two mice, and serum samples were collected, respectively. 20 µl of serum samples were mixed with an equal volume of 2× SDS non-reducing loading buffer, then diluted 20x with 1× SDS non-reducing loading buffer, and placed in water bath at 75°C for 5 min. The cleavage products were detected by SDS-PAGE.

Figure 13 shows the electrophoretogram of cleavage products resulting from cleavage of human IVIg by E97D_del18 at different times in the mice. The results showed that E97D_del18 effectively cleaved IVIg in the mice, and the enzymatic digestion was already substantially complete in 15 min.

### Example 13. Comparison of human IgG1-cleaving activity of combinatorial mutants

On the basis of the above-mentioned mutants, six combinatorial mutants were further constructed, the sequences of which are shown in Table 11.

**Table 11. Combinatorial mutants**

| Mutant | Modification relative to wild-type or mutant sequence | SEQ ID NO: |
|---|---|---|
| E97D_del18_delC5 | Deletion of the last 5 amino acids in SEQ ID NO: 28 | 30 |
| E97D_del18_delC10 | Deletion of the last 10 amino acids in SEQ ID NO: 28 | 31 |
| A59V_del18_delC5 | Deletion of the last 5 amino acids in SEQ ID NO: 27 | 32 |
| A59L_del18_delC5 | Deletion of the last 5 amino acids in SEQ ID NO: 26 | 33 |
| R280H_del18_delC5 | Deletion of the last 5 amino acids in SEQ ID NO: 29 | 34 |
| E97D_A59V_R280H | Combination of three mutations, E97D, A59V and R280H | 35 |

### 1. Expression and purification of mutants

According to the method in Example 1, the polynucleotide sequences of the mutants in Table 11 were synthesized, and the mutant expression recombinant plasmids were constructed to transform *Escherichia coli* BL21 Star (DE3). The mutant purified proteins were prepared according to the method in Example 4.

### 2. Comparison of human IgG1-cleaving activity of mutants

The purified mutants were diluted to 0.001 mg/mL, respectively. 50 ul of diluted mutants or wild-type IdeE was each added to 50 ul of reaction system containing 2 mg/ml of trastuzumab to initiate the cleavage reaction, and the reaction systems were placed at 37°C for 30 min. The samples were mixed with an equal volume of 2× SDS loading buffer, and then placed in water bath at 75°C for 5 min. The cleavage products were detected by SDS-PAGE.

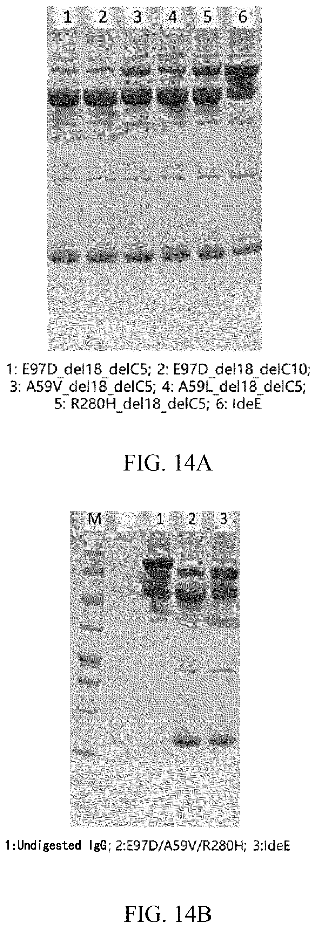

Figures 14A and 14B show the electrophoretograms of cleavage products resulting from cleavage of human IgG1 by the six combinatorial mutants (enzyme : substrate = 1 : 2000).

The Applicant declares that the detailed methods of the present invention are illustrated by means of the above-mentioned examples, but the present invention is not limited to the detailed methods mentioned above, that is, it does not mean that the present invention must rely on the detailed methods mentioned above to carry out. Those skilled in the art should understand that any improvement of the present invention, the equivalent replacement of each raw material of the articles of manufacture of the present invention, the addition of auxiliary components, the selection of specific methods, etc., fall within the scope of protection and the scope of disclosure of the present invention.

## Claims

1. A mutant of an immunoglobulin-degrading enzyme IdeE, wherein the immunoglobulin-degrading enzyme IdeE comprises or consists of an amino acid sequence as set forth in SEQ ID NO: 2 in the Sequence Listing; and the mutant comprises a mutation selected from the group consisting of:
(1) substitution of one or more of positions 8, 10, 24, 59, 97 and 280 of the amino acid sequence, thereby obtaining the mutant; and/or
(2) truncation of the immunoglobulin-degrading enzyme IdeE, by deleting the sequence of the first 1, the first 2, the first 3, the first 4, the first 5, the first 6, the first 7, the first 8, the first 9, the first 10, the first 11, the first 12, the first 13, the first 14, the first 15, the first 16, the first 17, the first 18 or the first 19 amino acids at its N-terminus; and/or
(3) truncation of the immunoglobulin-degrading enzyme IdeE, by deleting the sequence of the last 1, the last 2, the last 3, the last 4, the last 5, the last 6, the last 7, the last 8, the last 9 or the last 10 amino acids at its C-terminus;
wherein the mutant has higher activity than that of the immunoglobulin-degrading enzyme IdeE, and/or has higher thermal stability than that of the immunoglobulin-degrading enzyme IdeE.

2. The mutant according to claim 1, wherein the mutation is selected from the group consisting of:
(1) substitution of position 8, 10, 24, 59, 97 or 280 of the amino acid sequence as set forth in SEQ ID NO: 2; and/or
(2) deletion of the first 15, the first 16, the first 17, the first 18 or the first 19 amino acids at the N-terminus of the immunoglobulin-degrading enzyme IdeE, preferably the first 18 amino acids; and/or
(3) deletion of the last 5 or the last 10 amino acids at the C-terminus of the immunoglobulin-degrading enzyme IdeE, preferably the last 5 amino acids.

3. The mutant according to claim 1 or 2, wherein the substitution is selected from the group consisting of:
(1) the threonine at position 8 is substituted with any one of cysteine, phenylalanine, tryptophan, tyrosine, aspartic acid, glutamic acid, alanine, glycine, histidine, isoleucine, leucine, methionine, asparagine, proline, glutamine, serine, valine, arginine and lysine;
(2) the alanine at position 10 is substituted with any one of cysteine, aspartic acid, glutamic acid, phenylalanine, glycine, histidine, isoleucine, lysine, leucine, methionine, asparagine, proline, glutamine, arginine, serine, threonine, valine, tryptophan and tyrosine;
(3) the threonine at position 24 is substituted with any one of alanine, cysteine, aspartic acid, asparagine, glutamic acid, phenylalanine, glycine, histidine, isoleucine, lysine, leucine, methionine, proline, glutamine, arginine, serine, valine, tryptophan and tyrosine;
(4) the alanine at position 59 is substituted with any one of cysteine, aspartic acid, glutamic acid, phenylalanine, glycine, histidine, isoleucine, lysine, leucine, methionine, asparagine, proline, glutamine, arginine, serine, threonine, valine, tryptophan and tyrosine;
(5) the glutamic acid at position 97 is substituted with any one of alanine, cysteine, aspartic acid, phenylalanine, glycine, histidine, isoleucine, lysine, leucine, methionine, asparagine, proline, glutamine, arginine, serine, threonine, valine, tryptophan and tyrosine; and
(6) the arginine at position 280 is substituted with any one of alanine, aspartic acid, glutamic acid, cysteine, serine, phenylalanine, histidine, isoleucine, lysine, leucine, methionine, asparagine, proline, glutamine, tryptophan, threonine, valine and tyrosine.

4. The mutant according to claim 3, wherein the mutant is as set forth in any one of SEQ ID NOs: 3-35 in the Sequence Listing.

5. A protein, comprising the mutant according to any one of claims 1-4.

6. The protein according to claim 5, wherein the protein comprises a signal peptide at the N-terminus of the mutant; preferably, the protein is linked to a secretory signal sequence at the N-terminus of the mutant, and to methionine at the N-terminus of the secretory sequence, and/or is linked to a histidine tag at the C-terminus of the mutant; more preferably, the protein consists of the following from the N-terminus to the C-terminus: methionine, the secretory signal sequence and the mutant.

7. A nucleotide encoding the mutant according to any one of claims 1-4 or the protein according to claim 5 or 6.

8. An expression vector, comprising the nucleotide according to claim 7.

9. A host cell, comprising the expression vector according to claim 8 or expressing the mutant according to any one of claims 1-4 or the protein according to claim 5 or 6, preferably an *E. coli* cell or a yeast cell.

10. A composition, comprising:
an immunoglobulin-degrading enzyme or a mutant thereof or a protein comprising the immunoglobulin-degrading enzyme or the mutant thereof; and
optionally, a pharmaceutically acceptable carrier or excipient.

11. The composition according to claim 10, wherein the immunoglobulin-degrading enzyme is selected from IdeE, IdeS and IdeZ.

12. The composition according to claim 10 or 11, wherein the mutant of the immunoglobulin-degrading enzyme is the mutant according to any one of claims 1-4, or the protein comprising the immunoglobulin-degrading enzyme or the mutant thereof is the protein according to claim 5 or 6.

13. The composition according to any one of claims 10-12, further comprising:
an antibody or an Fc-containing protein.

14. The composition according to claim 13, wherein the antibody target is selected from the group consisting of: a cell surface protein, a cytokine, a hormone, an enzyme, an intracellular messenger, an intercellular messenger and an immune checkpoint.

15. The composition according to any one of claims 10-14, further comprising:
a viral vector drug, preferably selected from the group consisting of: an oncolytic virus, a gene therapy virus and a viral vector vaccine.

16. The composition according to any one of claims 10-15, further comprising:
an agent capable of reducing the IgG level in the blood, preferably selected from the group consisting of: an FcRn antibody and an Fc fragment variant with a high affinity to FcRn.

17. A kit, comprising:
(1) the mutant according to any one of claims 1-4 or the protein according to claim 5 or 6; and
(2) one or more selected from the group consisting of: (a) a pharmaceutically acceptable carrier or excipient; and (b) an antibody or an Fc-containing protein; and/or
(3) a viral vector drug selected from an oncolytic virus, a gene therapy virus and a viral vector vaccine; and/or
(4) an agent capable of reducing the IgG level in the blood selected from an FcRn antibody and an Fc fragment variant with a high affinity to FcRn.

18. A kit, comprising: a part A and a part B, wherein
the part A comprises the mutant according to any one of claims 1-4 or the protein according to claim 5 or 6, and
the part B comprises one or more selected from the group consisting of:
(1) a pharmaceutically acceptable carrier or excipient; (2) an antibody or an Fc-containing protein; and/or (3) a viral vector drug; and/or (4) an agent capable of reducing the IgG level in the blood;
wherein the viral vector drug is selected from an oncolytic virus, a gene therapy virus and a viral vector vaccine; and the agent capable of reducing the IgG level in the blood is selected from an FcRn antibody and an Fc fragment variant with a high affinity to FcRn.
